# EUROPEAN PATENT SPECIFICATION

(11) **EP 1 352 623 B1**
(45) Date of publication and mention of the grant of the patent: **26.11.2014**
(21) Application number: 03009955.0
(22) Date of filing: 26.10.1999
(51) Int. Cl.: A61F 9/008, A61B 3/18

(54) **Apparatus for determining and ablating corneal tissue volume necessary for correcting a visual ametropia**
Gerät zur Bestimmung und zur Ablation von Korneagewebe um Fehlsichtigkeiten zu korrigieren
Dispositif pour la détermination et pour l'ablation de la cornea pour corriger l'ametropia d'un oeil

(30) Priority: 12.07.1999 IT MI991526
(43) Date of publication of application: 15.10.2003
(62) Divisional of application: 99954353.1
(73) Proprietor: IVIS TECHNOLOGIES S.r.l, 74100 Taranto (IT)
(72) Inventor: D' Ippolito, Giuseppe, 74100 Taranto (IT)
(74) Representative: Wegner, Hans

(56) References cited:
- WO-A-98/42291
- DE-A- 4 337 842
- US-A- 4 721 379
- US-A- 5 571 107
- US-A- 5 740 815

## Description

### BACKGROUND OF THE INVENTION

The present invention relates to an apparatus for determining and ablating the corneal tissue volume necessary for correcting visual ametropia, as optimized for each individual patient.

As is known, for correcting visual ametropia, it is necessary to perform a corneal tissue ablation, and optimize it depending on the clinic status of each individual patient.

Document WO 98/42291 relates to a method for determining data for treating a surface, e.g. for treating a cornea by means of a laser. It discloses that the positions of a range of points on said surface are detected, e.g. by topometry, and fed into a computer. Moreover, a control surface is fed into the computer. The control surface may be determined based on clinical experience and/or raytracing. Then, the spacing between said points and the control surface are calculated by the computer. Finally, the position and spacing of the range of points are output as a data record for treating said surface. By means of the data record for treating said surface, a surface treatment method is simulated, and a result may be displayed to a surgeon. Different treatment methods and preset control surfaces and the respectively attainable result may be simulated. Based thereon, the surgeon may select the best treatment method.

### SUMMARY OF THE INVENTION

The aim of the present invention is to provide an apparatus adapted to mutually coordinate a set of operating devices, so as to univocally define the position, area and volume of the corneal structure to be ablated, for consequently performing the related processing.

Within the scope of the above mentioned aim, a main object of the present invention is to provide such an apparatus which is very efficient from an operating standpoint and which is specifically designed for providing accurate values to properly perform the ablating operation.

Yet another object of the present invention is to provide such an apparatus which, owing to its constructional peculiar features, is very reliable and safe in operation.

Yet another object of the present invention is to provide such an apparatus which can be easily made starting from easily available elements and materials and which, moreover, is very competitive from a mere economic standpoint.

According to one aspect of the present invention, the above mentioned aim and objects, as well as yet other objects, which will become more apparent hereinafter are achieved by an apparatus for determining and ablating a corneal tissue volume necessary for correcting a visual ametropia as defined in claim 1.

### BRIEF DESCRIPTION OF THE DRAWINGS

Further characteristics and advantages of the present invention will become more apparent hereinafter from the following detailed disclosure of a preferred, though not exclusive, embodiment of an apparatus for determining and ablating a corneal tissue volume necessary for correcting a visual ametropia, and being illustrated, by way of an indicative, but not limitative example, in the accompanying drawings, where:
Figure 1 illustrates an operating flow diagram, associated with the apparatus according to the present invention; and
Figure 2 illustrates an operating block diagram of the apparatus according to the present invention.

### DESCRIPTION OF THE PREFERRED EMBODIMENTS

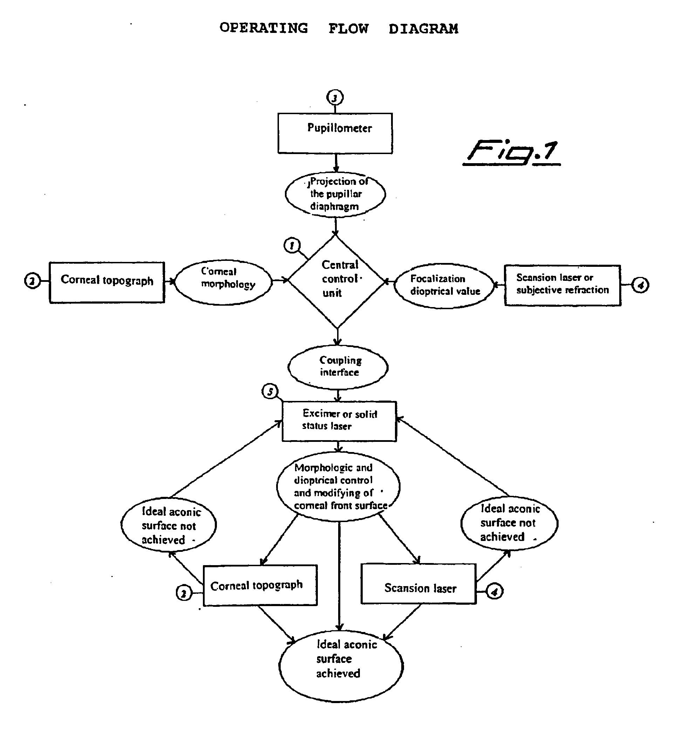

With reference to the number references of Figure 1, an apparatus for determining and ablating a corneal tissue volume necessary for correcting a visual ametropia, comprises a central control unit, generally indicated by the reference number 1, to which is connected a corneal topograph 2, operating for morphologically determining the front surface of the cornea.

To said unit 1 is connected an infrared pupillometer 3, for determining the iris diaphragm projection under scotopic conditions, at the level of the front corneal surface.

To said central unit is moreover connected a scansion laser 4 operating to define the dioptrical corrective value to be applied, point by point, with a discrete type of function, to the front corneal surface.

This value is determined by searching the diopter value optimizing the focalization at foveal level of each single light beam impinging on the corneal region, as in turn generated by a light source arranged at an infinite distance.

To said control unit 1 is moreover connected an excimer or solid status laser, of a microspot type, 5, having a coupling interface, for reading-out the altimetric ablative datum as expressed in micrometers on a square matrix in the x, y plane.

The apparatus according to the present invention allows to determine the ablating volume obtained by a crossing of the front corneal surface and an ideal aconic surface, as determined at a level of the pupillar diaphragm projection, as detected in a scotopic condition.

The front corneal surface is given by the corneal topograph 2.

In an embodiment which is not in all aspects in accordance with the invention, the ideal aconic surface is that surface determining the minimum of the sum function, as expressed in an absolute value, of the eccentricity, as measured in a radial direction, between the projection of the light beam impinging on the retina and the foveal center.

The light beam inciding on the retina is generated by a light source arranged at an infinite distance.

The sum or summation function analyzes, by discrete steps, all of the points where the impinging light beams cross the corneal front surface at an area delimited by the projection of the pupillar diaphragm as detected in a scotopic condition.

The connecting area between the ideal aconic surface and the residual corneal front surface is determined by assuring a constant curvature variation in a radial direction.

During the practical operation of the apparatus, an operator will detect, at the start, the corneal front surface by using said topograph 2.

Then, the operator will define the localization of said surface by locating the processing center as well as the reference axis.

The processing center, in particular, can be constituted by the corneal apex, the pupillar centroid projection, the fixation reflex or it can be selected at will by the operator.

The reference axis can comprise the optical axis, or the TV camera axis, or an axis perpendicular to the processing center.

The optical zone is delimited by the crossing perimeter of the corneal front surface, as determined by the topograph and localized as stated, and the preset ideal aconic surface.

The altimetric dislocation or offset between the two mentioned surfaces will be defined by determining the specific crossing perimeter which is circumscribed to the diameter referred to a projection on the corneal front surface of the pupillar diaphragm as detected in a scotopic condition and by the infrared pupillometer 3.

This function assures that the processing be performed through the overall corneal portion affected by the refractive process, by specifically limiting the overall volume to be ablated to a minimum necessary volume.

As the crossing perimeter exceeds the maximum diameter specified by the operator, it will be limited, for the exceeding portion, to the mentioned diameter.

According to the invention, said ideal aconic surface can be determined by a subjective refraction of the patient, as expressed in terms of correcting dioptrical values, either spherical and/or cylindrical, with the related asphericity axis and index.

In such a case, it is necessary to define the reference corneal refraction by determining that aconical surface adapted to better approximate the patient corneal front surface the center, axis and optical zone thereof being specified as previously stated.

The curvature, either maximum or minimum, of the preset aconical surface, referred to the corneal front surface refraction index, will express the related refractive power, in the term of the maximum and minimum values thereof.

That same aconical surface will express, moreover, the reference asphericity index.

In this latter case, the ideal aconic surface will be obtained from a vectorial summation of the refraction related to the aconic surface better approximating the corneal front surface, as determined as previously stated, and of the subjective refraction, as expressed as previously stated.

In particular, the operator pre-selects the surgical mode of operation, by choosing between PRK or LASIK, in order to properly correlate the processing amount and size, as designed at the level of the corneal front surface, depending on the actual distance thereof from the focal plane.

In particular, the operator preselects the target minimum width for the transition zone and the optional maximum ablation additional thickness, to obtain a constant curvature variation in a radial direction through the transition area, connecting the desired ideal aconic surface and residual corneal front surface.

Then, the operator will moreover define a maximum diameter for limiting therewithin the perimeter of the transition area.

The operator, in the case of a surgical PRK mode of operation, can possibly define, to obtain a laser corneal processing, the area thereon to add a constant thickness to be ablated, as defined with reference to the total processing perimeter, as well as the related value.

The above defined mode of operation, will establish univocally the processing mode both at a planimetric and at an altimetric level.

It has been graphically represented by different color areas, clearly showing the ideal aconic surface, the transition area between the ideal aconic surface and the residual corneal front surface, as well as the residual corneal front surface.

Moreover, by numeric data the operator will express or define the total ablation surface, the total ablation volume, the maximum ablation thickness and related planimetric dislocation as well as the ablation center planimetric dislocation with respect to the pupillar centroid.

The altimetric ablation datum, in particular, will be defined on a square matrix in the x, y plane, to allow the laser 5 to detect, through a suitable interface, the ablation profile or contour to be consequently performed.

As desired, the system, by intraoperatively detecting the morphologic datum and dioptrical variation will verify that the processing be carried out in accordance with the programmed ablation schedule, by modifying the latter, if necessary, together with the number of pulses localized for surface unit.

The processing is ended after having obtained a proper fitting of the detected and desired data.

From the above disclosure it should be apparent that the invention fully achieves the intended aim and objects.

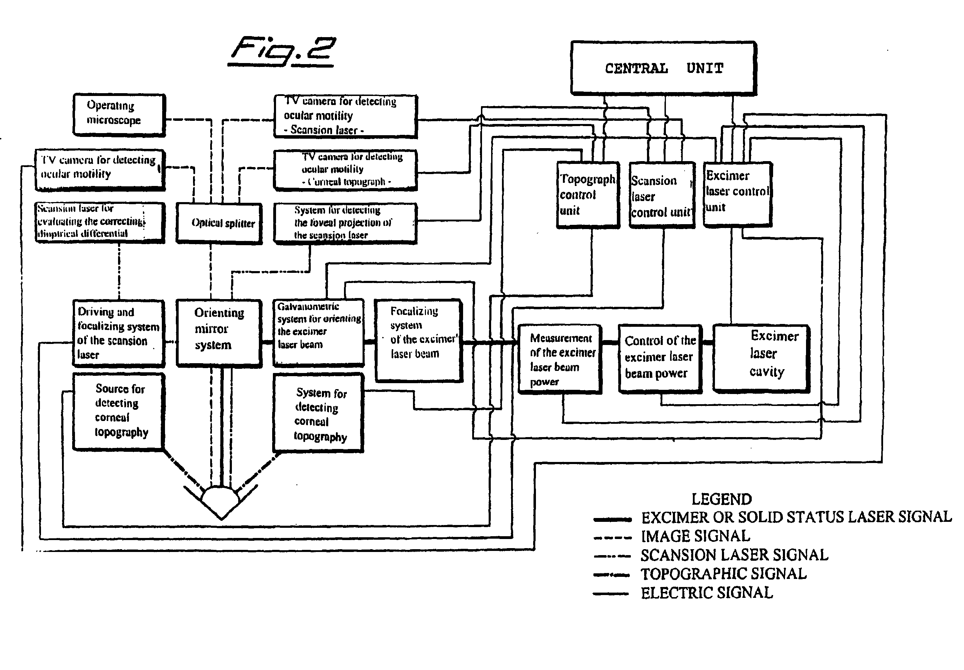

In particular, an apparatus according to the functional block diagram shown in Figure 2 has been provided, which apparatus is specifically adapted to provide very accurate and objective elements for controlling and performing the surgical operation.

The invention as disclosed is susceptible to several modifications and variations, all of which will come within the scope of the invention.

Moreover, all of the constructional details can be replaced by other technically equivalent elements.

In practicing the invention, the used materials, as well as the contingent size and shapes, can be any, depending on requirements.

## Claims

1. An apparatus for determining and ablating a corneal tissue volume necessary for correcting a visual ametropia, comprising:
a. a control unit (1) operatively connected to an excimer laser (5) or solid state laser (5), and to a corneal topographer (2), for morphologically determining a corneal front surface of an eye;
b. the apparatus being configured to:
b.1 determine an aconic surface adapted to approximate the corneal front surface of the eye;
b.2 determine a corneal ideal aconic surface from a vectorial summation of a refraction related to the determined aconic surface and of the subjective refraction of the eye; and
b.3 determine an ablating volume by a crossing of the corneal front surface of the eye and of the corneal ideal aconic surface.

2. Apparatus according to claim 1, wherein the refraction related to the determined aconic surface comprises at least a maximum refractive power and a minimum refractive power, and a first asphericity index.

3. Apparatus according to any of the foregoing claims, wherein the subjective refraction of the eye comprises at least a spherical correcting dioptrical value.

4. Apparatus according to any of the foregoing claims, wherein the subjective refraction of the eye further comprises a cylindrical correcting dioptrical value, and a cylinder axis.

5. Apparatus according to claim 3 or 4, wherein the subjective refraction of the eye further comprises a second asphericity index.

6. Apparatus according to any of the foregoing claims, wherein the apparatus is further configured to determine a connecting area between the corneal ideal aconic surface and a residual corneal front surface by assuring a constant curvature variation in a radial direction.

7. Apparatus according to any of the foregoing claims, further comprising an infrared pupillometer (3), for determining an iris diaphragm projection under scotopic conditions, at the level of the corneal front surface.

8. Apparatus according to claim 7, wherein the apparatus is further configured to determine a crossing perimeter of the corneal ideal aconic surface and the corneal front surface, which is circumscribed to a diameter on the corneal front surface of a pupillar diaphragm as detected in a scotopic condition.

9. Apparatus according to any of the foregoing claims, further comprising a coupling interface, for coupling said excimer or solid state laser (5) to said control unit (1).

10. Apparatus according to any of the foregoing claims, wherein the apparatus is further configured to end the ablation processing after having obtained intraoperatively a proper fitting of morphological data detected by the corneal topograph (2) and of the desired corneal ideal aconic surface.

## Patentansprüche

1. Vorrichtung zum Bestimmen und Abtragen eines Volumens eines Hornhautgewebes, nötig um eine optische Fehlsichtigkeit zu korrigieren, aufweisend:
a. eine Steuerungseinheit (1), die operativ mit einem Excimer-Laser (5) oder einem Festkörper-Laser (5), und mit einem Hornhaut-Topographen (2), zum morphologischen Bestimmen einer vorderen Hornhautoberfläche eines Auges, verbunden ist;
b. wobei die Vorrichtung eingerichtet ist zum:
b.1 Bestimmen einer akonischen Oberfläche, adaptiert um die vordere Hornhautoberfläche des Auges zu approximieren;
b.2 Bestimmen einer idealen akonischen Hornhautoberfläche aus einer vektoriellen Summierung einer Brechung, die mit der bestimmten akonischen Oberfläche in Beziehung steht, und einer subjektiven Brechung des Auges; und
b.3 Bestimmen eines Abtragungsvolumens durch ein Kreuzen der vorderen Hornhautoberfläche des Auges und der idealen akonischen Hornhautoberfläche.

2. Vorrichtung nach Anspruch 1, wobei die Brechung, die mit der bestimmten akonischen Oberfläche in Beziehung steht, zumindest eine maximale Brechkraft und eine minimale Brechkraft, und einen ersten Asphärizitätsindex aufweist.

3. Vorrichtung nach einem der vorhergehenden Ansprüche, wobei die subjektive Brechung des Auges zumindest einen sphärischen Korrekturdioptrienwert aufweist.

4. Vorrichtung nach einem der vorhergehenden Ansprüche, wobei die subjektive Brechung des Auges weiterhin einen zylindrischen Korrekturdioptrienwert und eine Zylinderachse aufweist.

5. Vorrichtung nach Anspruch 3 oder 4, wobei die subjektive Brechung des Auges weiterhin einen zweiten Asphärizitätsindex aufweist.

6. Vorrichtung nach einem der vorhergehenden Ansprüche, wobei die Vorrichtung weiterhin eingerichtet ist zum Bestimmen einer verbindenden Fläche zwischen der idealen akonischen Hornhautoberfläche und einer residuellen vorderen Hornhautoberfläche durch Sicherstellen einer konstanten Krümmungsvariation in eine radiale Richtung.

7. Vorrichtung nach einem der vorhergehenden Ansprüche, weiterhin aufweisend ein Infrarot-Pupillometer (3), zum Bestimmen einer Irisblenden-Projektion unter skotopischen Bedingungen, auf der Höhe der vorderen Hornhautoberfläche.

8. Vorrichtung nach Anspruch 7, wobei die Vorrichtung weiterhin eingerichtet ist zum Bestimmen eines Kreuzungsumfangs der idealen akonischen Hornhautoberfläche und der vorderen Hornhautoberfläche, der einem Durchmesser einer Pupillen-Blende, wie sie in einer skotopischen Bedingung detektiert wurde, umschrieben ist.

9. Vorrichtung nach einem der vorhergehenden Ansprüche, weiterhin aufweisend eine Kopplungsschnittstelle zum Koppeln des Excimer- oder Festkörper-Lasers (5) an die Steuerungseinheit (1).

10. Vorrichtung nach einem der vorhergehenden Ansprüche, wobei die Vorrichtung weiterhin eingerichtet ist, die Abtragungsbearbeitung zu beenden, nachdem intraoperativ eine korrekte Übereinstimmung morphologischer Daten, die vom Hornhaut-Topographen (2) detektiert wurden, und der gewünschten idealen akonischen Hornhautoberfläche erzielt worden ist.

## Revendications

1. Un dispositif pour la détermination et l'ablation d'un volume de tissu cornéen nécessaire pour corriger une amétropie visuelle, comprenant :
a. une unité de contrôle (1) reliée de manière opérante à un laser à excimère (5) ou un laser à état solide (5), et à un topographeur cornéen (2), pour la détermination morphologique d'une surface frontale cornéenne d'un oeil ;
b. le dispositif étant configuré pour :
b1. déterminer une surface aconique apte à approximer la surface frontale cornéenne de l'oeil ;
b2. déterminer une surface aconique idéale cornéenne à partir d'une somme vectorielle d'une réfraction relative à la surface aconique déterminée et de la réfraction subjective de l'oeil ; et
b3. déterminer un volume d'ablation par un croisement de la surface frontale cornéenne de l'oeil avec la surface aconique idéale cornéenne.

2. Dispositif selon la revendication 1, dans lequel la réfraction en relation avec la surface aconique déterminée comprend au moins une puissance réfractive maximale et une puissance réfractive minimale, et un premier indice d'asphéricité.

3. Dispositif selon l'une des revendications précédentes, dans lequel la réfraction subjective de l'oeil comprend au moins une valeur dioptrique de correction sphérique.

4. Dispositif selon l'une des revendications précédentes, dans lequel la réfraction subjective de l'oeil comprend en outre une valeur dioptrique de correction cylindrique, et un axe de cylindre.

5. Dispositif selon la revendication 3 ou 4, dans lequel la réfraction subjective de l'oeil comprend en outre un second indice d'asphéricité.

6. Dispositif selon l'une des revendications précédentes, dans lequel le dispositif est en outre configuré pour déterminer une zone de raccordement entre la surface aconique idéale cornéenne et une surface frontale cornéenne résiduelle en assurant une variation à courbure constante dans une direction radiale.

7. Dispositif selon l'une des revendications précédentes, comprenant en outre un pupillomètre à infrarouges (3), pour déterminer une projection du diaphragme de l'iris sous conditions scotopiques, au niveau de la surface frontale cornéenne.

8. Dispositif selon la revendication 7, dans lequel le dispositif est en outre configuré pour déterminer un périmètre de croisement de la surface aconique idéale cornéenne et de la surface frontale cornéenne, qui est circonscrit par un diamètre de la surface frontale cornéenne d'un diaphragme pupillaire tel que détecté dans une condition scotopique.

9. Dispositif selon l'une des revendications précédentes, comprenant en outre une interface de couplage, pour coupler ledit laser à excimère ou à état solide (5) à ladite unité de contrôle (1).

10. Dispositif selon l'une des revendications précédentes, dans lequel le dispositif est en outre configuré pour achever le processus d'ablation après avoir obtenu de manière intraopérante un ajustement approprié des données morphologiques détectées par le topographeur cornéen (2) et de la surface aconique idéale cornéenne souhaitée.
